Europäisches Patentamt

(19) European Patent Office

Office européen des brevets

(11) Publication number: **0 125 894**
**B1**

(12) **EUROPEAN PATENT SPECIFICATION**

(45) Date of publication of patent specification: **03.02.88**

(21) Application number: **84303173.3**

(22) Date of filing: **10.05.84**

(51) Int. Cl.⁴: **A 23 K 1/16,** A 23 L 1/30,
C 07 G 13/00

(54) Protection of vitamins.

(30) Priority: **14.05.83 GB 8313370**

(43) Date of publication of application:
**21.11.84 Bulletin 84/47**

(45) Publication of the grant of the patent:
**03.02.88 Bulletin 88/05**

(84) Designated Contracting States:
**BE CH DE FR GB IT LI NL**

(56) References cited:
**GB-A-1 435 550**
**US-A-2 401 293**
**US-A-2 855 306**
**US-A-3 992 555**
**US-A-4 182 778**

(73) Proprietor: **BP NUTRITION (UK) LIMITED**
**Stepfield Witham**
**Essex, CM8 3AB (GB)**

(72) Inventor: **Perry, Frederick George**
**BP Nutrition (UK) Limited Stepfield**
**Witham Essex, CM8 3AB (GB)**
Inventor: **Spence, Jeffrey Norman**
**BP Nutrition (UK) Limited (Norhtwich) Minsal**
**Works**
**Wincham Northwich Cheshire, CW9 6DF (GB)**

(74) Representative: **Crack, Richard David et al**
**BP INTERNATIONAL LIMITED Patents Division**
**Chertsey Road**
**Sunbury-on-Thames Middlesex TW16 7LN (GB)**

Courier Press, Leamington Spa, England.

# 0 125 894

**Description**

The present invention relates to the protection of stabilised vitamins in feedstuffs, particularly animal feedstuffs.

It is well known that the vitamin potency of feedstuffs, e.g. the vitamin A potency, falls during storage and that the rate of loss of potency is influenced by many factors such as the processing conditions under which the feedstuff is prepared, the temperature and humidity under which it is stored and, particularly, the presence of other ingredients in the feedstuff such as salt, trace minerals and moisture. It is further known that the loss of vitamin potency can be to some extent reduced by stabilising droplets of vitamin material in a matrix, for example gelatine, and coating the resulting beadlets with starch or some other similar dusting material. Such vitamin beadlets or particles are sometimes referred to as stabilised vitamins and will be referred to as such throughout the present specification. An alternative name sometimes used in the animal feedstuffs industry is encapsulated vitamins.

Stabilised vitamins frequently contain mixtures of vitamins e.g. vitamin A, vitamin D3 and vitamin E, all of which can also lose their potency on storage.

British Patent Specification 1,435,550 describes a method for producing an additive for animal feedstuffs, the additive containing vitamins which are to be, or which may be, subjected to moisture, the method comprising the steps of providing vitamin materials embedded in globules of gelatine (an example of a stabilised vitamin) and then mixing these globules with a toxicologically acceptable light oil before any mixing with water or moisture containing material to produce an additive in which the said vitamins are protected against degradation by moisture.

With a light oil it is difficult to ensure an overall coating; the oil may simply be absorbed without forming an effective coating. The particles may also be sticky and have a tendency to clump together.

Other nutrients may be included with the vitamins, particularly free access minerals, and the present invention includes stabilised vitamins containing such other nutrients.

The term "free access minerals" includes one or more of the following:— limestone, dicalcium phosphate, calcined magnesite, and trace elements such as copper salts, manganese salts and zinc salts. Other nutrients may also be present with the minerals, e.g. molasses for palatability and dust reduction. Free flow agents may also be present as well as waterproofing additives.

An object of the present invention is to provide a method for further improving the stability of stabilised vitamins particularly if they are to be compounded with moisture-containing materials.

Accordingly, the present invention provides a process for further improving the stability of stabilised vitamins which comprises mixing stabilised vitamin particles with a lipid in the solid state so that the lipid becomes adsorbed on the surfaces of the stabilised vitamin particles. The present invention further provides a process for the production of animal feedstuff additives which comprises mixing stabilised vitamin particles with a lipid in the solid state so that the lipid becomes adsorbed on the surfaces of the particles and incorporating said treated particles in an animal feed supplement.

The present invention includes stabilised vitamin particles comprising a core of stabilised vitamin and a surface coating of lipid. The present invention also includes an animal feedstuff additive containing lipid-coated, stabilised vitamin particles.

It has been proposed to coat various nutrient particles with lipids for various purposes. However, stabilised vitamins, as their name implies, have already been given a protective treatment, e.g. encapsulation in gelatine and, as far as is known, it has not been previously propsed to coat such stabilised vitamins with lipid, the closest prior art being coating them with a light oil as disclosed above.

An important aspect of the present invention is that the stabilised vitamins are coated with lipid in the solid state. Coating stabilised vitamins is not a simple task. Being susceptible to moisture, they cannot be coated by deposition or precipitation from an aqueous solution. Being heat sensitive they cannot be coated by using a heated molten source of coating material. Application of the coating in the solid state using the particular technique described may be the only practicable method.

The stabilised vitamin particles may contain a single vitamin or mixtures of two or more vitamins. They may also, as indicated above, contain minerals.

The vitamin stability of any of the known and commercially available stabilised vitamins can be enhanced according to the present invention. Stabilised vitamin A is particularly prone to loss of vitamin potency and can therefore be usually treated according to the present invention.

The term "lipid" means fats, waxes and their related products. In particular this term includes:
Fatty acids or salts thereof
Fatty alcohols or fatty acid esters thereof
Glycerol esters of fatty acids (e.g. triglycerides) or complex esters thereof
Phosphatides, e.g. lecithin
Mixtures of the above.

The lipid should, as indicated above, be solid at ambient temperature and preferbaly has a melting point of at least 50°C. More particularly it should have a melting point in excess of any temperature it is likely to be subjected to in the subsequent processing of the lipid-coated stabilised vitamin. Thus the lipid coated stabilised vitamin may be mixed with other feed components and pelleted. Temperatures in pelleting presses may reach 80°C and the lipid preferably has a melting point in excess of that temperature.

2

**0 125 894**

The melting point of the lipid may also affect the ease with which it is adsorbed onto the stabilised vitamin particles. Thus if the lipid chosen is too soft there is a tendency for the mixture of lipid and stabilised vitamin to clump and make further mixing difficult. Similarly if the lipid is too hard it is difficult to ensure that it is adequately adsorbed on the surface of the vitamin particles. A suitable upper limit for melting point may be 150°C.

Preferred lipids are fatty acids or fatty acid salts. When using either fatty acids or their salts the fatty acid may have from 14 to 24 carbon atoms. Either single-fatty acids or mixtures may be used and they may conveniently be in comminuted or beadlet form.

Fatty acids are frequently available commercially as mixtures, possibly with other related compounds, and a suitable lipid is one having a high content of free fatty acid, desirably at least 50% and preferably at least 90% wt.

A suitable source of acids is the material produced by Unichema International and sold under the name Dairy Fat Prills by BP Nutrition (UK) Limited. Dairy Fat Prills have a melting point of 51°C minimum and a free fatty acid content of 99% (85% being saturated acids and the fatty acid being predominantly $C_{16}$ and $C_{18}$ acids).

Fatty acid salts may be preferred to fatty acids since the salts will normally have higher melting points than the acids themselves, and it is to be understood that the above comments on preferred fatty acids apply equally to their salts.

Preferred salts are alkali or alkaline earth metal salts, the term alkaline earth metal including magnesium. A particularly preferred fatty acid salt is calcium stearate.

Another preferred type of lipid is hydrogenated fat or fat which is sufficiently hard naturally to be solid and able to be produced in particulate or beadlet form.

The amount of lipid should obviously be sufficient to coat the stabilised vitamin and further protect it from degradation. The upper limit will be determined by practical considerations and a convenient range may be from 2.5 to 30% wt. by weight of the coated particles. The use of lipid in an amount in excess of that required for simple coating may not be disadvantageous since lipids are animal feed components in themselves. Further the coating of lipid may act as a lubricant in subsequent processing steps. In particular it may act as a lubricant in a subsequent pelleting step and help to keep down the temperature rise which normally occurs during pelleting. Some vitamins may be degraded or destroyed by high temperatures so keeping the temperature down could be important.

The method by which the stabilised vitamins and the lipids are mixed can be varied according to the equipment available. Adequate mixing is indicated by the disappearance of the solid lipid. It is preferred to add the lipid to the mixing equipment in particulate form. In a preferred method the stabilised vitamin particles are ground with particles of the lipid in a mill. It is important not to subject the stabilised vitamin to too much shear because this appears to cause some reduction in the potency of the vitamins. With a given mixture and given apparatus, control experiments are preferably carried out to determine optimum mixing conditions. It is preferred to carry out the mixing at room temperature.

Particularly suitable equipment for use on a commercial scale is a Melanger mill. This is a slowly rotating granite basin which is steam heated, if need be, from the base. Sitting in the basin are two large granite wheels which can be adjusted for clearance from the base. These wheels are not powered but are rotated only by the momentum of the rotating basin. Situated between the wheels on the base of the basin are two plough shares which turn over the product being processed in order to give it a mixing action.

Stabilised vitamins with solid lipids adsorbed thereon according to the present invention find many uses in animal feedstuff technology. For example a vitamin mineral supplement may be prepared by mixing the necessary supplementary minerals with the improved stabilised vitamins according to the present invention, or by coating a stabilised vitamin already containing minerals. Such a supplement can then be mixed in a conventional manner with the known major components of animal feedstuffs e.g. cereals. Typically animal feedstuffs may contain from 0.1 to 10% of a supplement, e.g. a vitamin mineral supplement as described above.

A particular advantage of the present invention is that the stability of vitamins in any final product in which the improved stabilised vitamins have been incorporated, is greatly increased, and as a result, the potency of the product, i.e. feedstuff or supplement can be guaranteed for a greater period of time. Smaller quantities of vitamins can be employed in feedstuffs, or supplements can be produced having a guaranteed minimum vitamin content after a given period of time under specified storage conditions. This can lead to substantial cost savings.

The present invention is illustrated by the following examples.

Example 1

8 parts by weight of a gelatine encapsulated vitamin (Roche vitamin A/D3 in powder form and having the texture of a fine sand) and 2 parts by weight of a particulate commercial stearic acid (Unichema Pristerene 4691) were gently ground together by pestle in a mortar until visible signs of the stearic acid particles disappeared.

The same grinding process was also carried out on the encapsulated vitamin alone.

Samples of the two ground materials and of the original encapsulated vitamin were separately mixed

3

with a destructive medium consisting of salts, i.e., sodium chloride and copper, ferrous and manganese sulphates.

The mixed samples were then placed in dry dishes into a desiccator containing some water, sealed and placed in an incubator at 40°C.

Vitamin A analyses were carried out at the start and then after 2, 4 and 7 days of incubation.

The results shown in Table 1 below illustrate that the vitamins treated with the stearic acid according to the present invention were very much more resistant to the destructive conditions than the untreated material.

TABLE 1

|  | Control | | Ground control | | Protected vitamin and stearic acid | |
|---|---|---|---|---|---|---|
|  | Vit. A (iu/g) | % loss | Vit. A (iu/g) | % loss | Vit. A (iu/g) | % loss |
| Start | 531,245 | — | 542,000 | — | 441,300 | — |
| Day 2 | 526,029 | 1.0 | 526,000 | 3.0 | 441,300 | — |
| Day 4 | 424,560 | 20.1 | 432,740 | 20.2 | 407,350 | 7.7 |
| Day 7 | 220,480 | 58.5 | 228,960 | 57.8 | 373,380 | 15.4 |

The experimental error in the analyses was estimated to be ±5%.

Example 2

Three samples prepared as in Example 1 were further tested in a destructive medium consisting of
426 g calcined ferrous sulphate
600 g copper sulphate (FX grade)
200 g Choline Dry mix 50% cereal base
1044 g maize meal.

The samples were mixed into the above medium at a level of 6 miu Vit. A/5 lbs. to form simulated feed supplements which were then incubated at 37°C in sealed bottles. Vitamin A determinations were then carried out at the start and then after 4, 7 and 14 days of incubation.

The results shown in Table 2 below again show the increased protection against Vitamin A destruction obtained by the treatment process according to the process of the present invention.

TABLE 2

|  | Control | Ground | Ground and stearic acid |
|---|---|---|---|
| % loss Vit. A After 4 days | 5.4 | 15.4 | Nil |
| After 7 days | 47.1 | 35.2 | 16.6 |
| After 14 days | 71.6 | 66.3 | 59.2 |

Example 3

Trials were carried out to investigate the effect of varying the type of commercial stearic acid employed, the level of inclusion and the grinding time to give optimum protection to encapsulated vitamins. (Roche Vitamin A/D3).

Attempts were made to produce twenty seven samples given all permutations of the variables listed below:

Stearic acid type

Three commercial materials were used as supplied by Unichema: details are given in the table

| Trade Name | M.P.°C | Composition | | | | |
|---|---|---|---|---|---|---|
| | | $C_{12}/C_{14}$ | $C_{16}$ | $C_{18}$ | $C_{18\ 1}$ | Unsaturated acid |
| Pristerene 4934 | 50 min | ———————————— variable ———————————— | | | | |
| Pristerene 4961 | 52.5 min. | 3 | 48 | 44 | 5 | 8 |
| Pristerene 4916 | 54—56 | 4 | 39 | 54 | 2 | 1 |

Grinding time
    (a) 3 mins.
    (b) 6 mins.
    (c) 9 mins.

Level of stearic acid inclusion
    (a) 10%
    (b) 20%
    (c) 30%

All sample mixtures were produced by gentle grinding in a mortar at room temperature, and then, along with untreated control samples, were mixed into the destructive medium as described in Example 2.

Only 18 samples were eventually produced for testing since mixing became difficult over 6 minutes, particularly with 30% levels of stearic acid.

Vitamin A determinations were carried out immediately after mixing with the destructive medium and then again after 7 and 14 days incubation at 37°C.

The results (Table 3) show that all treatments improved the stability of vitamin A over the control samples under the destructive conditions of the trial, increased stability being less marked after 14 days than 7 days.

The use of 30% stearic acid did not generally afford any greater stability than the 10 and 20% levels. Results between mixing times vary—but it appears that little if any extra protection has been gained by grinding samples for longer than 3 minutes.

From physical aspects Pristerene 4916 appeared to be the most suitable stearic acid since, being harder than Pristerene 4961 and very much harder than Pristerene 4934, it presented fewer problems during mixing with the encapsulated vitamins and produced a freer flowing product.

# 0 125 894

TABLE 3

| Pristerene | Addition (%) | Mixing time (mins.) | Potency loss (%) | |
|---|---|---|---|---|
| | | | 7 days | 14 days |
| 4961 | 10 | 3 | Nil | 18.0 |
| | " | 6 | 10.1 | Nil |
| | 20 | 3 | 6.5 | 15.6 |
| | " | 6 | 3.6 | 9.6 |
| | 30 | 3 | 9.7 | 9.7 |
| | " | 6 | 13.4 | 15.1 |
| 4934 | 10 | 3 | 4.5 | 13.9 |
| | " | 6 | 3.0 | 11.6 |
| | 20 | 3 | Nil | 12.2 |
| | " | 6 | Nil | 22.8 |
| | 30 | 3 | 10.5 | 24.9 |
| 4916 | 10 | 3 | 6.3 | 14.0 |
| | " | 6 | 4.2 | 7.0 |
| | " | 9 | Nil | 20.1 |
| | 20 | 3 | 14.0 | 16.3 |
| | " | 6 | 4.5 | 17.3 |
| | 30 | 3 | Nil | 23.2 |
| | " | 6 | 7.7 | 15.6 |
| Control samples: | | | 18.3 | 28.2 |
| | | | 12.3 | 27.3 |
| | | | 24.1 | 31.9 |

Example 4

First trial

25 kg of encapsulated Roche Vitamin A/D$_3$ (500 000 iu/g Vitamin A and 100 000 iu/g Vitamin D$_3$) were poured into a Melanger basin, together with 5 kg of Dairy Fat Prills. The Dairy Fat Prills had the following characteristics and chemical composition:—

| | |
|---|---|
| Setting point | 52°C |
| Free fatty acids | 99% |
| Iodine value | 12 |
| Saponification value | 207 |
| G.L.C. analysis: | |
| $C_{12}$—$C_{14}$ | 2% |
| $C_{16}$ | 45% |
| $C_{17}$ | 2% |
| $C_{18}$ | 38% |
| $C_{18}'$ | 12% |
| $C_{20}$ | 1% |

The Melanger was set in motion and the height of the granite wheels adjusted to give a small amount of pressure to the mix in the basin. A series of samples were taken as follows:—

Sample 1 Initial sample of Vitamin A/D$_3$.

Sample 2 Sample of mix after 6 minutes.

Sample 3 Sample of mix after 12 minutes.

Sample 4 Sample of mix after 20 minutes.

Second trial

This was a repeat of the first trial, but reducing the level of Dairy Fat Prills. Thus 25 kg of Vitamin A/D$_3$

6

were added, followed by 2.5 kg of Dairy Fat Prills in two stages, one half being added immediately and the remainder 2 minutes later.

Samples taken were:—

Sample 5   Sample of mix after 6 minutes
Sample 6   Sample of mix after 10 minutes
Sample 7   Sample of mix after 15 minutes
Sample 8   Sample of mix after 20 minutes
Sample 9   Sample of mix after 30 minutes (with steam applied for last 10 minutes)
Sample 10   Sample of mix after 40 minutes (with steam applied for last 20 minutes)

The rotation speed of the Melanger basin was 20 rpm and this gave good mixing. A small build up of solid acid was noted on the base of the basin at the end of the first trial. This was scraped away before the start of the second trial.

In the second trial there was less acid on the base. At the end of the trials with Samples 9 and 10 steam was applied to the base of the mill in an attempt to prevent acid deposition, but no visible or physical effects were noted, presumably because the thickness of the granite basin and its low heat conductivity meant that there was no temperature rise inside the basin within the time scale used.

An accelerated storage trial was carried out on representative Samples. Untreated Vitamin A/D$_3$ from the same batch was used as a control and all samples were incorporated into a destructive medium at 5 miu per kilo, stored in an incubator at 39°C and assayed for Vitamin A at weekly intervals.

The composition of the destructive medium used for the accelerated storage trial was as follows:

25% Choline Chloride on silica (50% choline chloride/50% silica)
10% Copper sulphate
10% Ferrous sulphate
55% Limestone

Results (expressed as percentage retention of Vitamin A compared to theoretical)

| | Initial | 1st Week | 2nd Week | 3rd Week | 4th Week | 5th Week |
|---|---|---|---|---|---|---|
| Control | 100.8 | 94.0 | 98.2 | 85.4 | 82.4 | 69.8 |
| Sample | | | | | | |
| 3 (16.6% DFP mixed for 12 mins.) | 100.6 | 104.0 | 107.2 | 92.8 | 97.2 | 99.0 |
| 4 (16.6% DFP mixed for 20 mins.) | 100.0 | 101.6 | 103.0 | 95.4 | 89.4 | 89.8 |
| 6 (9% DFP mixed for 10 mins.) | 106.0 | 102.4 | 103.0 | 95.4 | 100.0 | 89.4 |
| 8 (9% DPF mixed for 20 mins.) | 101.2 | 101.4 | 100.4 | 94.4 | 98.2 | 89.0 |
| 9 (9% DFP mixed for 30 mins.*) | 99.0 | 105.3 | 106.8 | 87.4 | 90.6 | 73.8 |
| *steam for last 10 minutes) | | | | | | |

Conclusions

1. All treatments of coating Vitamin A/D$_3$ beadlets with Dairy Fat Prills at either 9% DFP or 16.6% DFP showed better potency retention than the control.
2. 9% DFP appears to produce satisfactory results.
3. Extension of mixing time beyond 10 minutes does not give any beneficial effect.
4. Application of steam does not have any benefit.

Example 5

Further accelerated storage trials were carried out on encapsulated vitamins as follows.

The encapsulated vitamins were the vitamin A/D3 beads of Example 4. Non-lipid coated vitamins were used as control and compared with the following lipid coated vitamins all prepared in a Melanger basin as described in Example 4.

Vitamin A/D3 coated with 10% wt. calcium stearate
Vitamin A/D3 coated with 20% wt. calcium stearate
Vitamin A/D3 coated with 9% wt. Dairy Fat Prills

The percentages were by weight of the encapsulated vitamin.

The calcium stearate was supplied by Durham Chemicals Limited. It had a melting point of 145°C.

The accelerated storage trials were carried out using the same medium and in the same way as described in Example 4.

The results obtained were:—

7

# 0 125 894

| | Initial | 2 weeks | 4 weeks | 6 weeks |
|---|---|---|---|---|
| Control—uncoated A/D$_3$ | 94.4 | 104.2 | 52.1 | 30.9 |
| 10% wt. Calcium stearate | 101.8 | 100.3 | 76.8 | 66.0 |
| 20% wt. Calcium stearate | 102.9 | 100.6 | 93.6 | 85.4 |
| % wt. Dairy Fat Prills | 103.7 | 101.7 | 99.1 | 87.1 |

The results show that:
(1) All coatings gave better potency retention than the control
(2) The 20% wt. treatment with calcium stearate gave better protection than the 10% wt. treatment
(3) The good results obtained with Dairy Fat Prills in Example 4 were confirmed.

## Claims

1. A process for further improving the stability of stabilised vitamins comprising mixing stabilised vitamin with a lipid in the solid state so that the lipid becomes adsorbed on the surfaces of the stabilised vitamin particles.

2. A process for the production of animal feedstuff additives comprising mixing stabilised vitamin particles with a lipid in the solid state so that the lipid becomes adsorbed on the surfaces of the particles and incorporating said treated particles in an animal feed supplement.

3. A process as claimed in Claim 1 or Claim 2 wherein the stabilised vitamin particles contain minerals.

4. A process as claimed in Claim 1, 2 or 3 wherein the lipid is one or more fatty acids or salts thereof.

5. A process as claimed in any of Claims 1 to 4 wherein the lipid has a melting point of at least 50°C.

6. A process as claimed in any of Claims 1 to 5 wherein the lipid content of the coated particles is from 2.5 to 30% wt.

7. A process as claimed in any of Claims 1 to 6 wherein the lipid and stablised vitamin particles are mixed by grinding in a mill.

8. A process as claimed in Claim 7 wherein the mill is a Melanger mill.

9. Stabilised vitamin particles comprising a core of stabilised vitamin and a surface coating of lipid.

10. An animal feedstuff additive containing lipid-coated stabilised vitamin particles as claimed in Claim 9.

11. A product as claimed in Claim 9 or 10 wherein the stabilised vitamin particles contain minerals.

12. A product as claimed in Claim 9, 10 or 11 wherein the lipid is one or more fatty acids or salts thereof.

13. A product as claimed in any of Claims 9 to 12 wherein the lipid has a melting point of at least 50°C.

14. A product as claimed in any of Claims 9 to 13 wherein the lipid content of the coated particles is from 2.5 to 30% wt.

15. An animal feedstuff containing from 0.1 to 10% wt. of an animal feedstuff additive as claimed in any of Claims 10 to 14.

## Patentansprüche

1. Verfahren zur weiteren Verbesserung der Stabilität stabilisierter Vitamine, umfassend das Vermischen des stabilisierten Vitamins mit einem Lipid im festen Zustand, so daß das Lipid auf den Oberflächen der stabilisierten Vitamin-Teilchen adsorbiert wird.

2. Verfahren zur Herstellung von Tierfutter-Zusatzstoffen, umfassend das Vermischen stabilisierter Vitamin-Teilchen mit einem Lipid im festen Zustand, so daß das Lipid auf den Oberflächen der stabilisierten Vitamin-Teilchen adsorbiert wird, und das Einarbeiten der genannten behandelten Teilchen in einen Tierfutter-Ergänzugsstoff.

3. Verfahren nach den Ansprüchen 1 oder 2, worin die stabilisierten Vitamin-Teilchen Mineralien enthalten.

4. Verfahren nach den Ansprüche 1, 2 oder 3, worin das Lipid eine oder mehrere Fettsäuren oder deren Salze ist.

5. Verfahren nach irgendeinem der Ansprüche 1 bis 4, worin das Lipid einen Schmelzpunkt von wenigstens 50°C hat.

6. Verfahren nach irgendeinem der Ansprüche 1 bis 5, worin der Lipid-Gehalt der beschichtezen Teilchen 2,5 bis 30 Gew.-% beträgt.

7. Verfahren nach irgendeinem der Ansprüche 1 bis 6, worin das Lipid und die stabilisierten Vitamin-Teilchen durch Vermahlen in einer Mühle miteinander vermischt werden.

8. Verfahren nach Anspruch 7, worin die Mühle eine Melanger-Mühle ist.

9. Stabilisierte Vitamin-Teilchen, umfassend einen Kern aus stabilisiertem Vitamin und eine Oberflächen-Beschichtung aus Lipid.

10. Tierfutter-Zustatzstoff, enthaltend lipid-beschichtete stabilisierte Vitamin-Teilchen nach Anspruch 9.

11. Produkt nach den Ansprüchen 9 oder 10, worin die stabilisierten Vitamin-Teilchen Mineralien enthalten.

12. Produkt nach den Ansprüchen 9, 10 oder 11, worin das Lipid eine oder mehrere Fettsäuren oder deren Salze ist.

13. Produkt nach irgendeinem der Ansprüche 9 bis 12, worin das Lipid einen Schmelzpunkt von wenigstens 50°C hat.

14. Produkt nach irgendeinem der Ansprüche 9 bis 13, worin der Lipid-Gehalt der beschichteten Teilchen 2,5 bis 30 Gew.-% beträgt.

15. Tierfutter, enthaltend 0,1 bis 10 Gew.-% eines Tierfutter-Zusatzstoffes nach irgendeinem der Ansprüche 10 bis 14.

**Revendications**

1. Procédé pour améliorer encore davantage la stabilité des vitamines stabilisées, comprenant le mélangeage de la vitamine stabilisée avec un lipide à l'état solide de manière que le lipide s'adsorbe sur les surfaces des particules de vitamine stabilisée.

2. Procédé pour la production d'additifs pour aliments pour animaux, comprenant le mélangeage de particules de vitamine stabilisée avec un lipide à l'état solide, de façon que le lipide s'adsorbe sur les surfaces des particules, et l'incorporation desdites particules traitées dans un supplément pour aliments pour animaux.

3. Procédé tel que revendiqué à la revendication 1 ou 2, dans lequel les particules de vitamine stabilisée contiennent des matières minérales.

4. Procédé tel que revendiqué à la revendication 1, 2 ou 3, dans lequel le lipide est constitué par un ou plusieurs acides gras ou leurs sels.

5. Procédé tel que revendiqué dans l'une quelconque des revendications 1 à 4, dans lequel le lipide possède un point de fusion au moins égal à 50°C.

6. Procédé tel que revendiqué dans l'une quelconque des revendications 1 à 5, dans lequel la teneur en lipide des particuleas revêtues se situe entre 2,5 et 30% en poids.

7. Procédé tel que revendiqué dans l'une quelconque des revendications 1 à 6, dans lequel les particules de vitamine stabilisée et revêtues de lipide sont mélangées par broyage dans un broyeur.

8. Procédé tel que revendiqué à la revendication 7, dans lequel le broyeur est un broyeur "Melanger".

9. Particules de vitamine stbailisée, comprenant un noyau de vitamine stabilisée et un revêtement superficiel de lipide.

10. Additif pour aliments pour animaux, contenant des particules de vitamine stabilisée et revêtues de lipide, telles que revendiquées à la revendication 9.

11. Produit tel que revendiqué à la revendication 9 ou 10, dans lequel les particules de vitamine stabilisée contiennent des matières minérales.

12. Produit tel que revendiqué à la revendication 9, 10 ou 11, dans lequel le lipide est constitué par un ou plusieurs acides gras ou leurs sels.

13. Produit tel que revendiqué dans l'une quelconque des revendications 9 à 12, dans lequel le lipide possède un point de fusion au moins égal à 50°C.

14. Produit tel que revendiqué dans l'une quelconque des revendications 9 à 13, dans lequel la teneur en lipide des particules revêtues se situe entre 2,5 et 30% en poids.

15. Aliment pour animaux, contenant de 0,1 à 10% en poids d'un additif pour aliments pour animaux tel que revendiqué dans l'une quelconque des revendications 10 à 14.